(19) Europäisches Patentamt
European Patent Office
Office européen des brevets

(11) Veröffentlichungsnummer: **0 072 508**
**A2**

(12) # EUROPÄISCHE PATENTANMELDUNG

(21) Anmeldenummer: 82107108.1

(22) Anmeldetag: 06.08.82

(51) Int. Cl.³: **C 07 D 231/22**
//C09B29/50, C09B29/32, C09B43/00

(30) Priorität: 18.08.81 DE 3132608

(43) Veröffentlichungstag der Anmeldung: 23.02.83 Patentblatt 83/8

(84) Benannte Vertragsstaaten: CH DE FR GB LI

(71) Anmelder: BAYER AG
Zentralbereich Patente, Marken und Lizenzen
D-5090 Leverkusen 1, Bayerwerk(DE)

(72) Erfinder: Kunde, Klaus, Dr.
Uppersberg 45
D-5090 Leverkusen 1(DE)

(54) **Pyrazolonderivate, ihre Herstellung und ihre Verwendung als Kupplungskomponenten.**

(57) Verbindungen der allgemeinen Formel

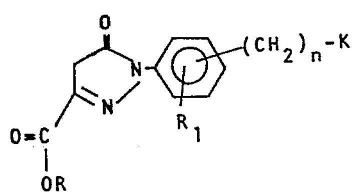

(1)

worin

R $C_1$-$C_4$-Alkyl,

$R_1$ Wasserstoff oder ein nichtionischer Substituent,

n 0, 1 oder 2,

K 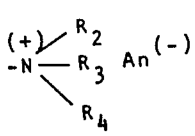 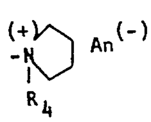 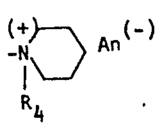

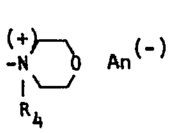 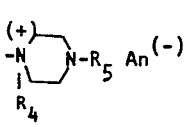 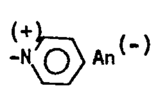

./...

EP 0 072 508 A2

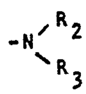 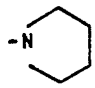 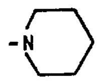 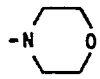 oder 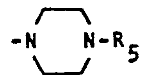

$R_2$ und $R_3$ unabhängig voneinander Wasserstoff, $C_1$-$C_4$-Alkyl, β-Hydroxy-$C_2$-$C_4$-alkyl oder Benzyl,

$R_4$ und $R_5$ unabhängig voneinander Wasserstoff, $C_1$-$C_4$-Alkyl oder β-Hydroxy-$C_2$-$C_4$-alkyl und

$An^{(-)}$ ein Anion sind,

werden als Kupplungskomponenten bei der Herstellung von Azofarbstoffen verwendet.

BAYER AKTIENGESELLSCHAFT 5090 Leverkusen, Bayerwerk

Zentralbereich
Patente, Marken und Lizenzen Mi/by-c

# Pyrazolonderivate, ihre Herstellung und ihre Verwendung als Kupplungskomponenten

Gegenstand der Erfindung sind Verbindungen der allgemeinen Formel

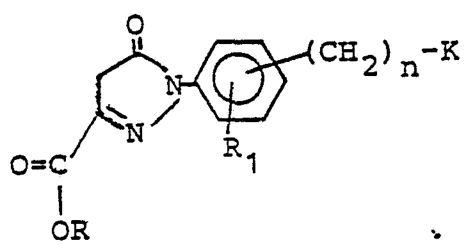

(I)

worin

R $C_1$-$C_4$-Alkyl

$R_1$ Wasserstoff oder ein nichtionischer Substituent,

n 0, 1 oder 2,

K 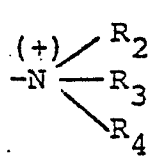 $An^{(-)}$ 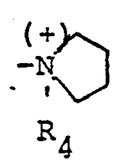 $An^{(-)}$ 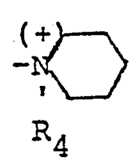 $An^{(-)}$

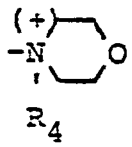 $An^{(-)}$ 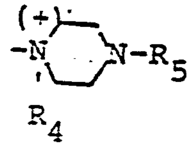 $An^{(-)}$ 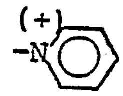 $An^{(-)}$

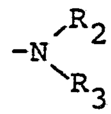 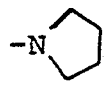 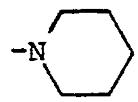 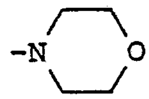 oder 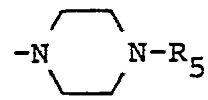

$R_2$ und $R_3$ unabhängig voneinander Wasserstoff, $C_1$-$C_4$-Alkyl, ß-Hydroxy-$C_2$-$C_4$-alkyl oder Benzyl,

$R_4$ und $R_5$ unabhängig voneinander Wasserstoff, $C_1$-$C_4$-Alkyl oder ß-Hydroxy-$C_2$-$C_4$-alkyl und

$An^{(-)}$ ein Anion sind,

ihre Herstellung und ihre Verwendung als Kupplungskomponenten.

Unter einem nichtionischen Substituent $R_1$ wird vorzugsweise Halogen, z.B. Fluor, Chlor oder Brom, $C_1$-$C_4$-Alkyl und $C_1$-$C_4$-Alkoxy verstanden.

$An^{(-)}$ kann ein Anion einer organischen oder einer anorganischen Säure darstellen. Bevorzugt sind Anionen, die bei der Herstellung der Verbindungen anfallen, z.B. bei der Quaternierung der Ausgangsverbindungen oder bei der Diazotierung. Als Beispiele seien Halogenide wie Chlorid oder Bromid, Sulfat, Bisulfat, Methosulfat, Benzolsulfat, Acetat oder Hydroxy genannt. Diese Anionen können in bekannter Weise gegen andere Anionen ausgetauscht werden.

Bevorzugt werden Verbindungen der allgemeinen Formel (I), worin

R Methyl oder Ethyl,

$R_1$ Wasserstoff, Methyl, Methoxy, Fluor, Chlor oder Brom,

n 0 oder 1,

K ein Rest der Formel 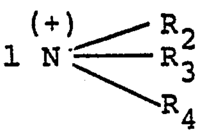 $An^{(-)}$

$R_2$ und $R_3$ unabhängig voneinander Methyl, Ethyl, 2-Hydroxyethyl oder 2-Hydroxypropyl,

$R_4$ Wasserstoff, Methyl, Ethyl, 2-Hydroxyethyl oder 2-Hydroxypropyl und

$An^{(-)}$ ein Anion sind.

Zur Herstellung der Verbindungen (I) werden Amine der allgemeinen Formel

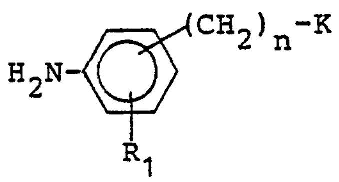

worin $R_1$, n und K die Bedeutung der Formel (I) besitzen, diazotiert, mit einer Verbindung der allgemeinen Formel

$$RO - CO - CH_2 - CH(COX) - CO - OR \qquad (III)$$

worin

X $C_1$-$C_4$-Alkyl, $C_1$-$C_4$-Alkoxy, Phenyl, Benzyl oder Wasserstoff ist, und

R die Bedeutung der Formel (I) besitzt,

gekuppelt und anschließend der Ring geschlossen.

Als Amine der allgemeinen Formel (II) können z.B. verwendet werden:

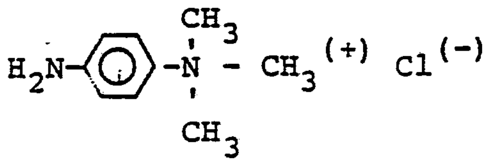

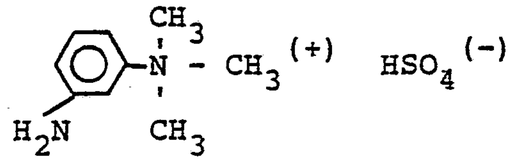

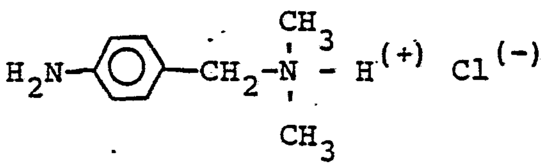

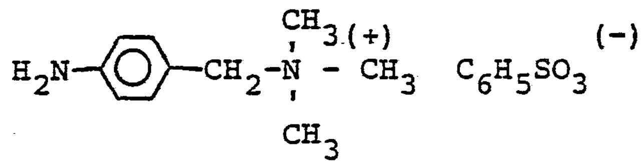

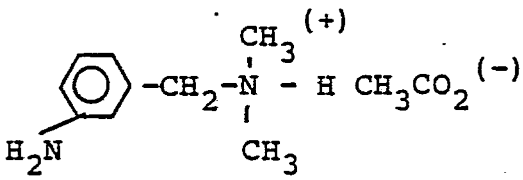

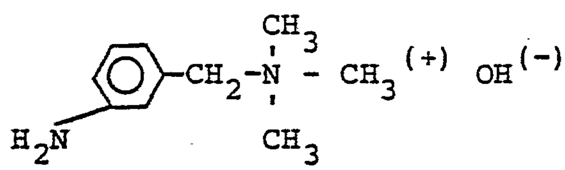

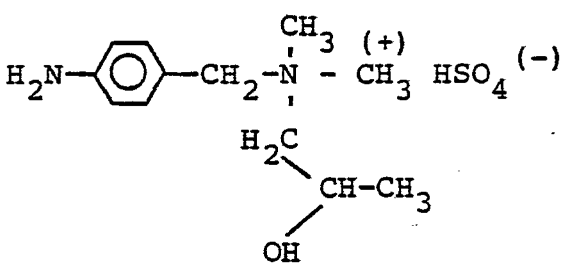

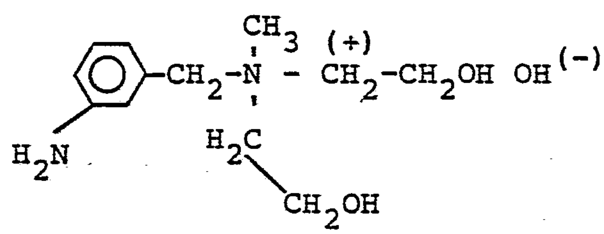

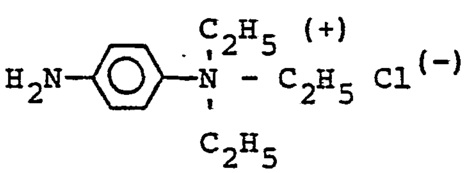

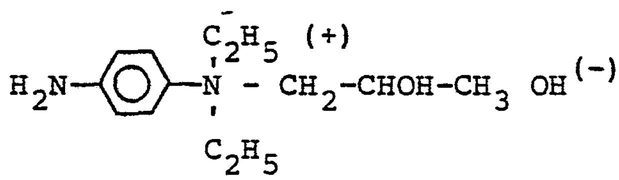

Dabei können auch die entsprechenden Salze anderer Säuren verwendet werden.

Als Kupplungskomponenten der allgemeinen Formel (III) können z.B. 2-Acetylbernsteinsäuredimethylester, 2-Acetylbernsteinsäurediethylester, 2-Formylbernsteinsäuredimethylester oder 2-Formylbernsteinsäurediethylester verwendet werden.

Das Verfahren zur Herstellung von Pyrazolonen, die keine kationische Gruppe enthalten, aus Diazoniumverbindungen und 2-Acylbernsteinsäureestern ist an sich bekannt. Es wird beispielsweise in JA 55 12 150 beschrieben. Es wurde nun gefunden, daß man auch kationische Amine der allgemeinen Formel (II) als Diazokomponenten verwenden kann. Die Kupplung erfolgt bei Temperaturen zwischen 0 und 50°C, vorzugsweise zwischen 0 und 30°C, bei pH-Werten zwischen 2 und 9, vorzugsweise zwischen 3 und 5. Der Ringschluß erfolgt bei Temperaturen zwischen 20 und 100°C, vorzugsweise bei Temperaturen zwischen 30 und 60°, bei pH-Werten zwischen 5 und 10, vorzugsweise zwischen 6 und 8. Anschließend wird gegebenenfalls durch Säurezugabe ein pH-Wert von $\leq 6$ eingestellt, um ein Verseifen der Carbonestergruppe zu vermeiden.

Der Verlauf der Reaktion läßt sich leicht chromatographisch verfolgen.

Die erfindungsgemäßen Verbindungen werden vorzugsweise ohne Zwischenisolierung zu Farbstoffen weiterverarbeitet, da sie gut wasserlöslich sind. Außerdem verlaufen Kupplung und Ringschluß mit sehr guten Ausbeuten. Man

kann die Verbindungen z.B. in der Weise isolieren, daß man die Reaktionslösung eindampft und die Verbindungen mit einem organischen Lösungsmittel, z.B. Ethanol oder Aceton, aus dem Rückstand extrahiert.

Beispiel 1

15 g 4-Aminobenzyldimethylamin werden mit 100 g Eis und 45 ml 30 %iger Salzsäure in 100 ml Wasser gelöst und durch Zugabe von 6,9 g Natriumnitrit diazotiert. Man gibt 18,8 g Acetylbernsteinsäuredimethylester zu und kuppelt durch langsame Zugabe von 5 g Natriumacetat und 8 g Natriumhydrogencarbonat. Nach Beendigung der Kupplung wird mit verdünnter Natronlauge neutralisiert und 3 Stunden auf 50°C erwärmt, wobei der Pyrazolring gebildet wird. Nach Beendigung des Ringschlusses stellt man durch Zugabe von Salzsäure einen pH-Wert von 5.5 ein. Die resultierende Lösung enthält das Produkt der Formel

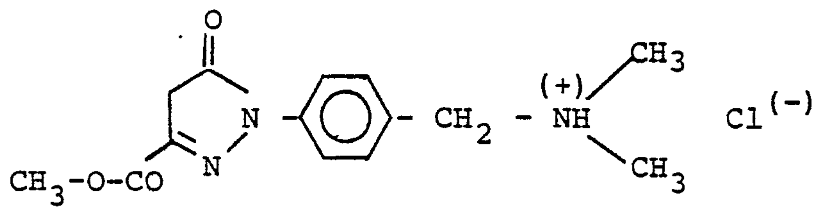

in 80 % Ausbeute.

Ersetzt man das 4-Aminobenzyldimethylamin durch eine äquimolare Menge 4-Aminophenyltrimethylammoniumhydrogensulfat, 3-Aminophenyltrimethylammoniumchlorid, 4-Aminobenzyltrimethylammoniumbenzolsulfonat oder 3-Aminobenzyltrimethylammoniumchlorid, so erhält man die entsprechenden Pyrazolone.

Beispiel 2

15 g 3-Aminobenzyldimethylamin werden mit 100 g Eis und 450 ml 30 %iger Salzsäure in 100 ml Wasser gelöst und durch Zugabe von 6,9 g Natriumnitrit diazotiert. Man gibt 17,4 g Formylbernsteinsäuredimethylester zu und kuppelt durch langsame Zugabe von 5 g Natriumacetat und 8 g Natriumhydrogencarbonat. Nach Beendigung der Kupplung wird mit verdünnter Natronlauge ein pH-Wert von 8,5 eingestellt und 1 Stunde auf 40°C erwärmt, wobei der Pyrazolring geschlossen wird. Nach Beendigung des Ringschlusses stellt man durch Zugabe von Salzsäure einen pH-Wert von 6 ein. Die resultierende Lösung enthält das Produkt der Formel

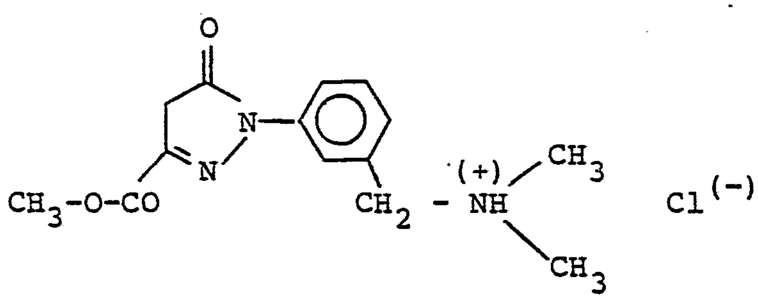

in 91 % Ausbeute.

Beispiel 3

6,7 g Anilin werden mit 20 ml 30 %iger Salzsäure und 100 g Eis in 100 ml Wasser gelöst und mit 5 g Natriumnitrit diazotiert. Man rührt 15 Minuten nach, zerstört Nitritüberschuß mit Amidosulfonsäure und gibt 33,5 g des nach Beispiel 1, Teil 1, erhaltenen Pyrazolons als

Acetat zu. Durch Zugabe von 20 %iger Natriumacetat-Lösung wird der pH-Wert zwischen 3,5 und 4 gebracht und solange gehalten, bis die Kupplung beendet ist. Der Farbstoff ist in Lösung. Er färbt Papier und Polyacrylnitril-Fasern in gelben Tönen.

Patentansprüche:

1. Pyrazolonderivate der allgemeinen Formel

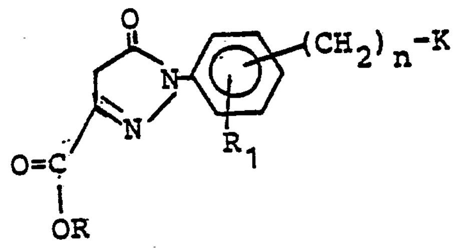

worin

R $C_1$-$C_4$-Alkyl,

$R_1$ Wasserstoff oder ein nichtionischer Substituent,

n 0, 1 oder 2

K 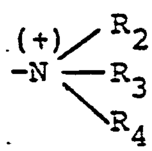 An$^{(-)}$ 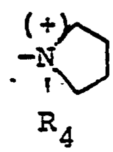 An$^{(-)}$ 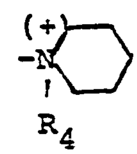 An$^{(-)}$

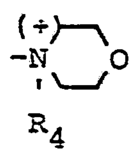 An$^{(-)}$ 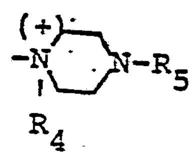 An$^{(-)}$ 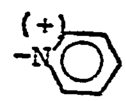 An$^{(-)}$

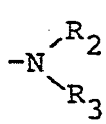 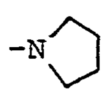 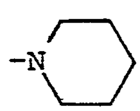 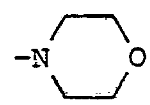 oder 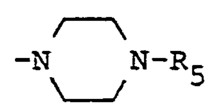

$R_2$ und $R_3$ unabhängig voneinander Wasserstoff, $C_1$-$C_4$-Alkyl, ß-Hydroxy-$C_2$-$C_4$-alkyl oder Benzyl,

$R_4$ und $R_5$ unabhängig voneinander Wasserstoff, $C_1$-$C_4$-Alkyl oder ß-Hydroxy-$C_2$-$C_4$-alkyl und

$An^{(-)}$ ein Anion sind.

2. Pyrazolonderivate der Formel des Anspruchs 1, worin

R Methyl oder Ethyl,

$R_1$ Wasserstoff, Methyl, Methoxy, Fluor, Chlor oder Brom,

n 0 oder 1,

K ein Rest der Formel 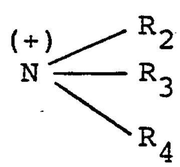 $An^{(-)}$

$R_2$ und $R_3$ unabhängig voneinander Methyl, Ethyl, 2-Hydroxyethyl oder 2-Hydroxypropyl,

$R_4$ Wasserstoff, Methyl, Ethyl, 2-Hydroxyethyl oder 2-Hydroxypropyl und

$An^{(-)}$ ein Anion sind.

3. Verfahren zur Herstellung von Pyrazolonderivaten des Anspruchs 1, dadurch gekennzeichnet, daß man Amine der allgemeinen Formel

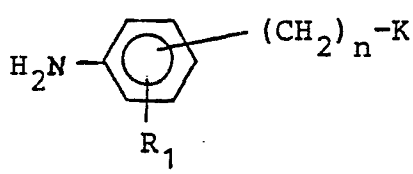

worin $R_1$, n und K die in Anspruch 1 angegebene Bedeutung besitzen, diazotiert, mit einer Verbindung der allgemeinen Formel

$$RO - CO - CH_2 - CH(COX) - CO - OR$$

worin

X $C_1$-$C_4$-Alkyl, $C_1$-$C_4$-Alkoxy, Phenyl, Benzyl oder Wasserstoff ist, und

R die in Anspruch 1 angegebene Bedeutung hat,

kuppelt und den Ring schließt.

4. Verwendung der Pyrazolonderivate des Anspruchs 1 als Kupplungskomponenten zur Herstellung von Azofarbstoffen.